# EUROPÄISCHE PATENTSCHRIFT

(11) **EP 2 798 382 B1**
(45) Veröffentlichungstag und Bekanntmachung des Hinweises auf die Patenterteilung: **27.04.2016**
(21) Anmeldenummer: 12816460.5
(22) Anmeldetag: 28.12.2012
(51) Int. Cl.: G01W 1/16

(54) **SELBSTAKTIVIERENDES ADAPTIVES MESSNETZ UND VERFAHREN ZUR REGISTRIERUNG SCHWACHER ELEKTROMAGNETISCHER SIGNALE**
SELF-ACTIVATING ADAPTIVE MEASURING NETWORK AND METHOD FOR REGISTERING WEAK ELECTROMAGNETIC SIGNALS
RÉSEAU DE MESURE ADAPTATIF AUTO-ACTIVABLE ET PROCÉDÉ D'ENREGISTREMENT DE SIGNAUX ÉLECTROMAGNÉTIQUES FAIBLES

(30) Priorität: 31.12.2011 DE 102011122807
(43) Veröffentlichungstag der Anmeldung: 05.11.2014
(73) Patentinhaber: Elwe Technik GmbH, 38162 Cremlingen (DE)
(72) Erfinder: PABST, Michael, J., 96231 Bad Staffelstein (DE)
(74) Vertreter: Dreykorn-Lindner, Werner
(86) Internationale Anmeldenummer: PCT/EP2012/005386
(87) Internationale Veröffentlichungsnummer: WO 2013/097946

(56) Entgegenhaltungen:
- EP-A2- 1 355 165
- EP-B1- 0 990 177
- DE-A1-102004 000 024
- DE-A1-102004 000 025

## Beschreibung

Die Erfindung betrifft, gemäß dem Patentanspruch 1, ein Messnetz zur Registrierung schwacher elektromagnetischer Signale und, gemäß dem Patentanspruch 9, ein Verfahren hierzu.

Spherics sind sehr kurz dauernde, oft nur aus wenigen Schwingungen bestehende gedämpfte, vertikal und horizontal polarisierte Schwingungen, die durch Ladungsverschiebungen oder Ladungswanderungen in der Troposphäre entstehen. Die Frequenzen liegen zwischen 3 und 100 kHz, die Amplituden beim bis zu 1000fachen normaler Funkübertragung, die Impulsfolgefrequenz liegt bei maximal 150 Hz und die typische Impulsdauer liegt zwischen 35 bis 250 µs. Hauptquellen für Spherics sind Gewitter, deren Blitzaktivitäten elektromagnetische Felder erzeugen, sowie luftelektrische Schwankungen, die durch die Bewegung und Reibung großer Luftmassen ausgelöst werden. Da es sich bei den verursachenden Blitzen oft um nicht sichtbare Dunkelfeldentladungen (ohne Donner) handelt, werden diese Spherics, d.h. das impulshafte Auftreten elektromagnetischer Wellen natürlichen Ursprungs innerhalb der Erdatmosphäre, oft auch als Dunkelblitze bezeichnet.

Die Aktivitäten von Spherics werden in der Meteorologie zur Wettervorhersage herangezogen, wobei mit Hilfe ihrer in digitalen Wetterstationen gewonnenen Daten die Wetterprognosen wesentlich verfeinert werden können. Für die Datenfernübertragung zu einer Zentrale kommen meist kabelgeburidene Übertragungswege oder über analoge-, ISDN- oder Funkmodems zum Einsatz. In modernen Wetterstationen werden zunehmend auch auf dem Internetprotokoll basierende Nachrichtenübertragungswege genutzt, wodurch Wetterdaten über standardisierte Protokolle in lokalen Netzwerken (local area network LAN) und Funknetzwerken (wireless local area network WLAN), aber auch weltweit über das Internet oder auch direkt als SMS verfügbar sind.

Aus der DE 10 2004 000 024 A1 ist ein System sowie ein Verfahren zum Erfassen, Übermitteln und Auswerten von durch elektromagnetische Strahlung anfallenden Daten und Informationen bekannt, bei dem die Sensitivität zur Erfassung der elektromagnetischen Strahlung möglichst hoch ist und gleichzeitig möglichst wenige Störsignale erfasst werden. Hierzu weist das System auf:
- mehrere Messstationen,
- mit jeweils mindestens einem insbesondere breitbandigen Antennenkörper zum Erfassen von der elektromagnetischen Strahlung zuordbaren Signalen,
- mit jeweils mindestens einer separat vom Antennenkörper angeordneten Stationselektronik zum Verarbeiten der mittels des jeweiligen Antennenkörpers erfassten Signale zu den Daten und Informationen sowie
- mit jeweils mindestens einer Speichereinheit zum Speichern der von der Stationselektronik verarbeiteten Daten und Informationen und
- mindestens eine Zentralstation, an die zumindest ein Teil, insbesondere mindestens ein ausgewählter Parameter, der von der jeweiligen Messstation erfassten, verarbeiteten und gespeicherten Daten und Informationen übermittelbar ist,
- mindestens eine selbstregulierende Schwelle, mittels derer die Sensitivität der Stationselektronik, insbesondere mindestens einer Filtereinheit der Stationselektronik, in Abhängigkeit vom auftretenden Rauschen, insbesondere vom statistischen Rauschpegel, einstellbar, insbesondere optimierbar, ist.

Im Einzelnen weist die Stationselektronik
- mindestens eine Verstärkereinheit zum rauscharmen Verstärken der mittels des jeweiligen Antennenkörpers erfassten Signale ,
- mindestens eine Filtereinheit zum Filtern der mittels der Verstärkereinheit verstärkten Signale, insbesondere im Hinblick auf zum Beispiel von Radiosendern stammende technische Störsignale, sowie
- mindestens eine Analog/Digital-Wandlereinheit (zum Wandeln der mittels der Filtereinheit gefilterten Signale in digitale Ausgangssignale auf.

Weiterhin ist mindestens ein Blockpuffer vorgesehen, der mit den Signalen, insbesondere mit den digitalen Ausgangssignalen der Analog/Digital-Wandlereinheit, beaufschlagbar ist und erforderlichenfalls für ein Zwischenspeichern dieser Signale, insbesondere dieser digitalen Ausgangssignale, ausgelegt ist. Mittels einer Ausleseeinheit wird ein fortlaufendes Abrufen der durch den Blockpuffer durchlaufenden und/oder erforderlichenfalls zwischengespeicherten Signale ermöglicht. Ferner ist der Ausleseeinheit ein Signalanalysemittel zum Analysieren und/oder zum Weiterverarbeiten der von der Ausleseeinheit ausgelesenen Ausgangssignale des Blockpuffers sowie zum insbesondere fortlaufenden Ermitteln des Rauschpegels nachgeschaltet. Schließlich ist eine Schwellenermittlungseinheit zum Ermitteln zumindest eines optimalen Schwellenwerts in Abhängigkeit vom ermittelten Rauschpegel und zum Beaufschlagen der selbstregulierenden Schwelle mit diesem ermittelten optimalen Schwellenwert eine Signalanalyseeinheit vorgesehen. Die Messstationen sind in einem Abstand von etwa einhundert Kilometern zueinander angeordnet und stehen jeweils in bidirektionaler, insbesondere drahtgebundener und/oder drahtloser Verbindung mit der Zentralstation. Der Antennenkörper ist in mechanisch selbsttragender Form ohne bewegliche und/oder witterungssensitive Komponenten zum Aufstellen im Freien ausgelegt, wobei der Primärkreis des Antennenkörpers vom Sekundärkreis des Antennenkörpers galvanisch getrennt ist und die elektromagnetischen Felder sind breitbandig und rauscharm auskoppelbar und damit zeitgetreu messbar. Die Zentralstation weist mindestens eine Auswerteeinheit auf, mittels derer die von den jeweiligen Messstationen übermittelten Daten und Informationen, insbesondere auch in hohen Raten und/oder insbesondere auch ohne zeitlichen Verlust, auswertbar sind, und weiterhin mindestens eine Warneinheit zum Ausgeben abgestufter und/oder vollautomatischer Warnungen vor klimatologischen und/oder meteorologischen Vorgängen, insbesondere vor Gewittern. Die übermittelten Daten und Informationen sind auf der Grundlage historischer, insbesondere mittels Langzeitmessung gewonnener Daten und Informationen mittels Model Output Statistics bewertbar. Schließlich ist mindestens eine, insbesondere der Zentralstation zugeordnete Schnittstelle zum Einbinden mindestens einer weiteren klimatologischen und/oder meteorologischen Daten- und Informationsquelle zur Charakterisierung der, insbesondere mittels Model Output Statistics bewertbaren Gesamtwettersituation vorgesehen. Das System gemäß der DE 10 2004 000 024 A 1 ermöglicht eine Erfassung schwacher echter Signale, denn die Signalschwelle wird automatisch in Abhängigkeit vom vorhandenen Rauschen auf den geringstmöglichen Wert eingeregelt. Die selbstregulierende Signalschwelle, die funktionell und/oder technisch von der totzeitfreien Erfassung und/oder vom parallelen Verarbeitungskreis unabhängig sein kann, ist hierbei üblicherweise nur bei zu hohem Datenanfall aktiv. Die selbstregulierende Schwelle beinhaltet dabei das adaptive Herausfinden der Rauschgrenze, was aufgrund statistischer Rauscheffekte, die beispielsweise durch die Unterschiede zwischen Tag- und Nachtgang und/oder durch Wettereffekte verursacht sind, erschwert wird. Die Eigenschaften und Funktionen der Zentrale, der bis zu einhundert Sensoren zugeordnet sein können, bestehen unter anderem:
- in der Signalspeicherung,
- in der Durchführung eines Verfahrens zur Ortung von Entladungsquellen, zum Beispiel von Blitzen, für klimatologische und/oder meteorologische Zwecke unter Einbeziehung extrem schwacher Signale aus schwachen atmosphärischen Entladungen (stille Entladung blitzartiges Ereignis) zur Optimierung der Früherkennung von Gewitterzellen,
- in der Implementierung von Modulen zur Online-Verarbeitung hoher Daten-/Informationsraten, wobei ein Paket aus Daten und Informationen eintrifft, und zwar in Einzelpakete zerlegt über fünfzehn Sekunden (Timestamp); eine logische Ringstruktur geht zu einer in der Zentraleinheit implementierten Auswerteeinheit (-> Software für Zentralstation zur Ortung und Auswertung von Blitzen), wodurch eine Peilung sowie Bewertung entsprechend einer zeitlichen Sortierung nach Stärke erfolgt (optimierte Anzahl von fünf bis sechs Sensoren),
- in der Durchführung eines Verfahrens zur Erkennung von Signalherden zur Früherkennung von Gewitterzellen,
- in der Durchführung eines Verfahrens zur Analyse von Typen von Signalen bzw. von Gruppen von Daten und Informationen zur Charakterisierung meteorologischer Klassen und demzufolge zur Erstellung von Blitzkarten,
- in der Durchführung eines Verfahrens zur dynamischen Extrapolation von räumlichen Zugbahnen erkannter Gewitterherde sowie
- in einem Modul zur vollautomatischen und gestuften Gewitterwarnung in Form von Text- und Warnmeldungen sowie deren Versand an Nutzer mittels einer Sendeeinheit.

Bei einer Weiterentwicklung hierzu, gemäß der DE 10 2004 000 025 A1, wird eine genaue Charakterisierung der Impulsquelle, zum Beispiel eine zuverlässige Unterscheidung zwischen Wolke-Boden-Blitzen (Cloud-Ground) und Wolke-Wolke-Blitzen (Intra-Cloud) innerhalb einer Wolke oder zwischen Wolken (Cloud-Cloud) ermöglicht. Hierzu weist das System zum Erfassen, Übermitteln und Auswerten von durch, insbesondere niederfrequente, elektromagnetische Strahlung anfallenden Daten und Informationen mehrere räumlich getrennte Messstationen mit jeweils mindestens einem insbesondere breitbandigen Antennenkörper zum Erfassen von der elektromagnetischen Strahlung zuordenbaren Signalen sowie mit jeweils mindestens einer Zeitmesseinrichtung, insbesondere mindestens einer Global-Positioning-System-Uhr, zum Ermitteln des jeweiligen zeitlichen Verlaufs, insbesondere der jeweiligen Ankunftszeit, der erfassten Signale auf. Die elektromagnetische Strahlung stammt aus mindestens einer Impulsquelle natürlichen und/oder nichtnatürlichen Ursprungs, insbesondere aus mindestens einer atmosphärischen Entladung bzw. von mindestens einem Sender. Dabei ist die Höhe der Impulsquelle, insbesondere die Emissionshöhe bzw. die Sendehöhe, und/oder die Direktionalität, insbesondere der räumliche Richtungsverlauf, der durch die Impulsquelle bewirkten Impulsabgabe oder Impulsaussendung lokalisierbar. Hierzu ist die Abweichung der Ankunftszeit des Signals an der der Impulsquelle nächstgelegenen Messstation von der Ankunftszeit des Signals an mindestens einer, vorzugsweise mindestens zwei, derselben Impulsquelle nicht nächstgelegenen Messstationen bestimmbar. Insbesondere bei linienförmigen Impulsquellen ist die Direktionalität der Impulsabgabe oder Impulsaussendung als im Wesentlichen vertikal identifizierbar, wenn sich die Amplitude des Signals reziprok proportional zur Entfernung der Impulsquelle von der jeweiligen Messstation verhält. Diese ist als im Wesentlichen horizontal identifizierbar, wenn die Amplitude des Signals von der reziproken Proportionalität abweicht, wobei diese Abweichung durch Berücksichtigung des Höhenwinkels und des Winkels zwischen Impulsabgabe- oder Impulsaussendungsachse, insbesondere Entladungsachse, und Richtung zur jeweiligen Messstation korrigierbar ist. Ferner sind mehrere, aus Impulsquellen eines räumlich und/oder zeitlich begrenzten Bereichs stammende Signale zu Gruppen zusammenfassbar. Die Abweichung der Amplitude eines einzelnen Signals ist zur insbesondere mittleren Abweichung der Amplitude der dem Signal zugeordneten Gruppe in Beziehung setzbar, insbesondere um einen durch variable Bodenleitfähigkeit bedingten Dämpfungseffekt zu eliminieren. Bei Impulsabgabe oder Impulsaussendung aus Höhen im Kilometerbereich ist mittels Vergleichen der an mindestens einer der Impulsquelle nächstgelegenen Messstation und an mindestens zwei derselben Impulsquelle nicht nächstgelegenen Messstationen gemessenen Verteilungen von Zeitabweichungen mindestens zweier Impulsabgabe- oder Impulsaussendungzeiten, insbesondere mindestens zweier Entladungszeiten, nachweisbar. Die Höhe der Impulsquelle und/oder die Direktionalität der Impulsabgabe oder Impulsaussendung ist mittels der der Impulsquelle nächstgelegenen Messstation sowie mittels einer einzigen, der Impulsquelle nicht nächstgelegenen Messstation bestimmbar, wenn die Einfallsrichtung der Impulsabgabe oder Impulsaussendung ermittelbar ist. Beim System gemäß der DE 10 2004 000 025 A1 erfolgt die Peilung nicht mit nur einer oder zwei der Impulsquelle nahen, insbesondere blitznahen, Sensoren auf direkte Weise, d.h. nicht durch ortsnahes Anpeilen in die Höhe, sondern durch Nutzung von Abweichungen der Signal-Ankunftszeiten an den jeweils der Impulsquelle nächstgelegenen, insbesondere blitznächsten, Sensorstationen gegenüber anderen, der Impulsquelle nicht nächstgelegenen, insbesondere nicht blitznächsten, Sensorstationen. Dabei werden in der Netzzentrale mittels besonderer Algorithmen, insbesondere mittels algorithmischer Mustererkennung, aus den zahlreichen, von den Einzelsensoren eintreffenden Signalen diejenigen herausgefunden, die zum gleichen Impulsereignis gehören. Zu diesem Zweck werden die von den verschiedenen Einzelsensoren empfangenen Peakmuster in digitalisierter Form "übereinander gelegt", um auf diese Weise den Anfangspeak eindeutig identifizieren zu können. Mittels dieser "Matching"-Methode ist ein zeitlich präzises und mithin gutes Peilergebnis erzielbar, was wiederum wesentlich für die Höhenpeilung ist. Hierdurch kann die hohe Ereignisempfindlichkeit erreicht und die dadurch begründete hohe Anzahl primär erfassbarer Signale tatsächlich weiter genutzt und auch hinsichtlich der Signaleigenschaften zweckdienlich ausgenutzt werden. Zur Berücksichtigung der Herkunft der Entladungen wird das Netz gemäß der DE 10 2004 000 025 A1 um einen dreidimensionalen Modus (sogenannter 3D-Modus) erweitert, so dass Emissionshöhen von Impulsen, insbesondere von Entladungen, ermittelt werden können. Eine derartige Vorgehensweise bietet eine Reihe von technischen wie auch wirtschaftlichen Vorteilen:
- weil nur eine Station impulsquellennah, insbesondere blitznah, sein muss, sind große Sensorabstände möglich, d.h. es ist kein dichtes teures Netz erforderlich;
- aufgrund der Nutzung Global-Positioning-System-gesteuerter Zeitmessungen können auch noch größere Entfernungen von bis zu etwa einhundert Kilometern zur Blitz-Station als "der Impulsquelle nah" bzw. als "blitznah" gelten und signifikante, d.h. für die Höhenpeilung nutzbare Zeitabweichungen produzieren;
- die Verwendung von Very Low Frequency-Messnetzen (= Bereich um zehn Kilohertz) sichert eine große Reichweite der der Impulsquelle nicht nächstgelegenen Messstationen, zum Beispiel der nicht blitznächsten Blitzstationen (die den Blitz auch noch erfassen müssen), d.h. etablierte VLF-Netze können eingesetzt werden, sofern die Zeitmessungen im Mikrosekundenbereich genau sind;
- das System wie auch das Verfahren kann auf der Hardware herkömmlicher 2D-Blitzortungsverfahren aufsetzen; eine Verwendung "echter" 3D-Techniken im Radio- oder VHF-Bereich ist insofern nicht erforderlich, als Ziel der vorliegenden Erfindung nicht die räumlich präzise Auflösung von Entladungskanälen, zum Beispiel von Blitzkanälen, im 100 Meter-Bereich, sondern die Bereitstellung einer Datenbasis für eine Entscheidung hinsichtlich der Frage ist, ob das detektierte Ereignis als Intra Cloud-Impuls einzustufen ist;
- die Existenz von Impulsabgaben bzw. Impulsaussendungen, insbesondere von Blitzemissionen, aus Kilometer-Höhen lässt sich theoriefrei und ohne Annahme justierbarer Parameter aus einem Vergleich der an einzelnen (jeweils blitznächsten) Sensoren und an Gruppen von (nicht blitznächsten) Sensoren gemessenen Verteilungen von Zeitabweichungen zweier Impulsabgabezeiten (im Netz durch Gesamtpeilung ermittelte Impulszeit minus Differenz von Ankunftszeit und Laufzeit an der jeweiligen der Impulsquelle nächstgelegenen Station), insbesondere zweier Blitzzeiten (im Netz durch Gesamtpeilung ermittelte Blitzzeit minus Differenz von Ankunftszeit und Laufzeit an der jeweiligen blitznächsten Station), erkennen;
- die Emissionshöhe lässt sich mit Algorithmen, die mit denen der Ortspeilung zumindest vergleichbar sind, durch Hinzufügen der Höhenvariablen berechnen;
- das Verfahren kann in andere existierende Impulsortungssysteme, insbesondere Blitzortungssysteme, integriert werden, sofern die notwendige Zeitgenauigkeit erzielt wird;
- das Verfahren kann auch bei intensitätsschwachen Impulsen, insbesondere bei intensitätsschwachen Blitzen, eingesetzt werden, sofern der Impuls, insbesondere der Blitz, an drei, bei Nutzung von Einfallsrichtungen zur Peilung auch an nur zwei Sensoren gemessen wird;
- die Ergebnisse des Verfahrens können dazu benutzt werden, Fragen einer Impulsform-Diskriminierung von Wolke-Wolke-Entladungen (IC), insbesondere von Wolke-Wolke-Blitzen, gegenüber Wolke-Erde-Entladungen (CG), insbesondere Wolke-Erde-Blitzen, zu klären und
- das System wie auch das Verfahren kann zusammen mit der Impulsform-Analyse zur Diskriminierung eingesetzt werden, um den (kleinen) Prozentsatz strittiger Fälle zu lösen, der weder mit dem einen Verfahren noch mit dem anderen Verfahren klar entscheidbar ist.

Weiterhin ist aus der EP 1 355 165 A2 ein Verfahren und eine Vorrichtung zur Erdbeben- sowie Vulkanausbruchsvorhersage und Epizentrumsortung mittels Sferics-Technics-Spektrumsanalyse bekannt. Um eine Sferics-Technics-Spektrumsanalyse zu schaffen, welche keinen niederfrequenten Signalempfänger wie schmalbandige Spulen und hierfür geeignete Verstärker und Analysatoren benötigt, werden atmosphärische, niederfrequente ELF-/VLF-Signale (ELF Extremely Low Frequency, VLF Very Low Frequency) bzw. elektromagnetische Felder in Gestalt von Sferics, Atmospherics, Technics über Antennenelemente erfasst, die erfassten ELF-/VLF-Signale bzw. elektromagnetischen Felder werden (echtzeit-)gefiltert, (echtzeit-)spektral analysiert, um zeitlich erstreckte, höchst niederfrequente ULF-Anomalien (Ein-/Umhüllende) zu ermitteln, und hieraus wird der Ursprung, vor allem die Richtung der ULF-Anomalie mittels mehrerer mit großem Abstand, vor allem mit einem Abstand von mehr als tausend Kilometer, angeordneter Empfänger-/Analyseeinheiten geortet. Dabei werden die erfassten ELF-/VLF-Signale bzw. elektromagnetischen Feldsignale sektoriell zur Ursprungs- bzw. Richtungsortung der ULF-Anomalie in ihre Richtungskomponenten zerlegt und in dieser Form analysiert. Die Vorrichtung ist gekennzeichnet durch mehrere, untereinander elektrisch und/oder elektromagnetisch verbundenen Sferics-Technics- bzw. Wetterstrahlungserfassungseinheiten und zugehörige Signalauswertungseinheiten, mindestens zwei auf der Erde entfernt aufgestellte Sferics-Technics-Wechselsignal-Erfassungssysteme mit jeweils mindestens drei Antennenelementen, die in jeweils senkrechter bzw. vertikaler, horizontaler vorwiegend Ost-West- sowie Nord-Süd-Richtung ausgerichtet sind, und den Erfassungssystemen nachgeschaltete SAT-Datenverarbeitungs-, ELF-/VLF-Erfassungs- und ULF-Untersuchungseinheiten. Die über Zuleitungselemente verknüpften Antennenelemente weisen eine Länge von zumindest einigen Dezimetern auf und bestehen aus einem elektrisch sowie elektromagnetisch leitfähigen Material. Der Sferics-Wechselsignalverstärker in den Erfassungssystemen umfasst breitbandig einen Frequenzbereich von einigen Zehn Kilohertz obere Grenzfrequenz bis kleiner/gleich einige Hertz und weist eine Signaldynamik von mindestens 50 dB auf. Insbesondere die für die Erfassung horizontaler Sferics-Technics-Aktivitäten zuständigen, senkrecht aufgestellten Antennen mit zugehörigen Antennensignalen und nachfolgender Sferics-Technics-Signalanalyse sind auf mehrere Richtungssektoren zu kleiner/gleich 90 Grad unterteilt. Dabei weisen die Antennen-Richtungssektoren zu je kleiner/gleich +/- 45 Grad um die Richtungen Nord, Süd, West, Ost eine Charakteristik-Keule auf. insbesondere sind niederfrequent hoch magnetisch leitende, also mit einer hohen Permeabilität versehene Metallelemente - vorzugsweise MU-Metall - um das Sferics-Technics-Signal-Antennenelement V-förmig, einen Winkel kleiner/gleich 90 Grad einschließend, gebildet. Schließlich sind die V-förmig ausgebildeten Richtungsselektionsantennen manuell und/oder motorgesteuert schwenk- bzw. drehbar aufgestellt.

Aus der EP 0 990 177 B1 der Anmelderin ist ein elektromagnetisches Messsystem für die Meteorologie bekannt, mit dem Spherics-Signale erfasst und analysiert werden ist. Bei diesem Messsystem, welches den Oberbegriffs des Patentanspruchs 1 bildet, wird von der Überlegung ausgegangen, dass die von einer Mehrzahl von Messstationen, die in Form eines Messstationsnetzes räumlich verteilt in einem vorgegebenen Raumgebiet angeordnet sind, eine Analyse der Veränderung, die ein Spherics-Signal in der Atmosphäre längs seiner Ausbreitung durch diese erfährt, einen besseren Aufschluss über das allgemeine Wettergeschehen ermöglicht. Insbesondere können durch eine feinmaschige und kontinuierlich erfolgende Messung von Spherics-Signalen neben den aktuellen Wettererscheinungen auch deren Ursachen, beispielsweise Luftbewegungen und Entladungsvorgänge, erfasst werden, so dass hinsichtlich der künftigen Wetterentwicklung eine insbesondere für kurzfristige Prognosen (typisch 15 Minuten bis 2 Stunden) hohe Treffsicherheit möglich ist. Grundidee bei diesem Messsystem der Anmelderin ist es somit, ein elektromagnetisches Messsystem für die Meteorologie einzurichten, dessen Sender durch stochastisch auftretende natürliche Ereignisse innerhalb der Atmosphäre dargestellt werden, und dessen Empfänger durch ein Netz von Messstationen gebildet werden. Weiterhin beruht dieses Messsystem auf der Überlegung, dass die Spherics-Signale längs ihres Ausbreitungsweges in der Atmosphäre durch lokal unterschiedliche thermodynamische und elektrische Gegebenheiten beeinflusst werden, und sich auf diese Weise ergänzende Rückschlüsse auf das aktuelle Wettergeschehen und die sich daraus ergebenden Folgerungen für eine Prognose ableiten lassen. Die von diesem Messsystem bereitgestellten Daten geben somit ein flächendeckendes Abbild der schwachen atmosphärischen VLF-Emissionen. Diese Daten können nicht nur als Grundlage für eine zuverlässige Kurzfrist-Wetterprognose, sondern darüber hinaus auch als verlässliche Grundlage für die epidemiologische Untersuchung der biotropen Wirkungen von VLF-Impulsen auf den menschlichen Organismus verwendet werden. Der mittlere Abstand benachbarter Messstationen, d. h. die Maschenweite des Messnetzgitters, beträgt vorzugsweise zwischen 10 km und 50 km, insbesondere etwa 30 km. Damit wird eine flächendeckende Erfassung der Spherics-Aktivitäten mit einer Auflösung von etwa 10 km ermöglicht. In einer bevorzugten Ausgestaltung des Messsystems ist jede Messstation mit einer Verarbeitungseinheit zum Ableiten von Messdaten aus den empfangenen Spherics-Signalen vorgesehen. Die Verarbeitungseinheit kann dabei aus einfachen Filtern und/oder analogen Signalbearbeitungsstufen bestehen, mit denen die Signale analog verarbeitet werden, so dass Messdaten in analoger Form anstehen. Insbesondere umfasst die Verarbeitungseinheit einen digitalen Signalprozessor, dem ein Analog-Digital-Wandler vorgeschaltet ist. In diesem Fall können die Spherics-Signale einer digitalen Signalanalyse, beispielsweise einer zusätzlichen digitalen Filterung, insbesondere einer Spektralanalyse oder einer Zeitreihenanalyse aufeinanderfolgender Spherics-Signale, unterzogen werden. Die vom Signalprozessor durch eine solche Analyse abgeleiteten Messdaten werden dann als digitales Datenwort an die zentrale Auswerteeinheit übermittelt.

Schließlich ist aus der DE 195 14 465 A 1 der Anmelderin ein einfacher, schneller und genauer Analysator für Spherics-Signale mit einem Signaleingang und einer Verarbeitungs- und Aufbereitungseinrichtung für eingehende Spherics-Signale sowie mit einem Ergebnissignalausgang bekannt, bei dem die Verarbeitungs- und Aufbereitungseinrichtung eine Fuzzy-Logik-Einheit enthält, welche zur unscharfen Bewertung der Spherics-Signale ausgelegt ist. Die Fuzzy-Logik-Einheit berücksichtigt zur unscharfen Bewertung der Spherics- Signale Signalkriterien, wie die Anstiegssteilheit, die Anfangsamplitude, die Schwingungsanzahl, die Frequenzverteilung, die Amplitudenverteilung und/oder das Abklingen der Schwingungen der Signale sowie die Impulsfolgefrequenz und die Häufigkeitsverteilung der auftretenden Frequenzwerte. Für jedes Kriterium sind Zugehörigkeitsfunktionen zur Fuzzifizierung bestimmt, und die Fuzzy-Logik-Einheit enthält eine Fuzzy-Logik-Schaltung zur Defuzzifizierung. Weiterhin ist die Fuzzy-Logik-Einheit ausgelegt, um anhand der Bewertung hinsichtlich des eingegangenen Signals eine Entscheidung herbeizuführen, ob es sich um ein Spherics-Signal oder eine Störungen handelt und/oder, ob das Signal aus dem Nah- oder Fernbereich stammt. Hierzu enthält die Verarbeitungs- und Aufbereitungseinrichtung einen der Fuzzy-Logik-Einheit vorgeschalteten Analog-Digital-Wandler und eine der Fuzzy-Logik-Einheit vorgeschaltete Speichereinrichtung zum Speichern von Eingangssignalen. In der Speichereinrichtung gespeicherte Signale sind zur Verarbeitung und Aufbereitung durch die Fuzzy-Logik-Einheit sequentiell aus der Speichereinrichtung abrufbar und der Fuzzy-Logik-Einheit zuführbar. Weiterhin ist zum Empfangen von Spherics-Signalen eine dem Signaleingang vorgeschaltete Antennenanordnung vorgesehen, welche richtungsselektiv und/oder für Magnetfeldschwankungen empfindlich ist und welche eine aktive Antenne mit einem Antennenkörper mit einem Vorverstärker und einem Impedanzwandler enthält. Weiterhin ist zwischen der Antennenanordnung und dem Signaleingang eine Verstärker- und Filtereinrichtung angeordnet, welche eine Filtereinheit aus SC-Filtern enthält und welche den Empfangsbereich des Analysators auf 3 kHz bis 60 kHz beschränkt. Vorzugsweise besteht die Fuzzy-Logik-Einheit aus einer zweiteiligen Logikschaltung, wobei der erste Teil der der Signalidentifizierung anhand vorgebbarer Parameter dient und der zweite Teil so ausgelegt ist, um eine Verknüpfung der Parameter miteinander auszuführen, so dass die zugehörigen Signale insbesondere eindeutig als Spherics-Signale erkennbar und von Störungen trennbar sind. Dabei sind Impulsdauern bis zu einigen 100 Mikrosekunden, Schwingungsfrequenzen zwischen 3 kHz und 100 kHz, insbesondere zwischen 3 kHz und 60 kHz, Impulsfolgefrequenzen zwischen null kHz und 150 Hz und maximale Signalamplituden im Bereich von einigen mV/m bis einigen V/m mess- und verarbeitbar.

Gegenstand der vorliegenden Erfindung ist ein Messnetz und ein Verfahren zur Registrierung schwacher elektromagnetischer Signale (Entladungskanal im Bereich 10¹ und 10² m, Stromstärke im Bereich 10¹ Ampere), die als Spherics-Bursts bezeichnet werden und insbesondere in konvektiven Zellen entstehen und sich deutlich von typischen Blitzen (Entladungskanal 10³m, Entladungsstromstärke 10³ Ampere) unterscheiden. Durch eine zuverlässige Detektion dieser Spherics-Bursts (Dunkelblitz und kaum hörbarer Donner) könnte die örtliche Vorhersage hinsichtlich der Wahrscheinlichkeit des bevorstehenden Auftretens von Blitzen/Blitzeinschlägen wesentlich verbessert werden.

Der Entstehungsmechanismus dieser schwachen Spherics-Burstsignale lässt sich wie folgt erklären: Ladungscluster, die bereits in frühen Entwicklungsstadien einer konvektiven Zelle entstehen, können sich impulshaft, mit den daraus resultierenden Impulsformen und Frequenzanteilen, durch eine Verbindung über sogenannte "Hydrometeore" (Eisnadeln, die sich im elektrischen Feld ausrichten und ein Filament bilden) entladen.

Der Erfindung liegt gegenüber den bekannten Messnetzen und Verfahren für die Meteorologie die Aufgabe zugrunde, diese derart auszugestalten, dass die Entstehung, Entwicklung und Verlagerung von Entladungsvorgängen relativ zum flächendeckenden Messnetz beobachtbar ist.

Diese Aufgabe wird, ausgehend von einem Messnetz zur Registrierung schwacher elektromagnetischer Signale mit den Merkmalen im Oberbegriff des Patentanspruchs 1, dadurch gelöst, dass dem Basisnetz ein Subnetz unterlagert ist, dass in Zellen des Subnetzes Sensoren mit einem im Verhältnis zwischen 1 : 8 und 1 : 12, vorzugsweise 1 : 10, kleineren Abstand verteilt angeordnet sind, dass die im Standby-Betrieb befindlichen Sensoren des Basisnetzes beim Empfang von in einem einstellbaren Zeitraum eintreffenden Spherics-Burstsignalen selbstaktivierend sind und eine Vorverarbeitung zur Gewinnung von Ereignismustern vornehmen und dass die Zentrale zur feineren Auflösung bezüglich der Beobachtung, die Sensoren des Subnetzes einschaltet.

Weiterhin wird diese Aufgabe, gemäß Patentanspruch 9, durch ein Verfahren zur Registrierung schwacher elektromagnetischer Signale in einem Messnetz mit einem Basisnetz mit Sensoren und einem dem Basisnetz unterlagerten Subnetz mit Sensoren gelöst, indem
- die im Standby-Betrieb befindlichen Sensoren des Basisnetzes beim Empfang von in einem einstellbaren Zeitraum eintreffenden Spherics-Burstsignalen selbstaktivierend sind,
- die Sensoren des Basisnetzes eine Vorverarbeitung zur Gewinnung von Ereignismustern vornehmen und
- die Zentrale zur feineren Auflösung bezüglich der Beobachtung, die Sensoren des Subnetzes einschaltet.

Das erfindungsgemäße Messnetz bzw. Verfahren richtet sich also nicht wie beim Stand der Technik, insbesondere der DE 10 2004 000 024 A1, der DE 10 2004 000 025 A1 und der EP 1 355 165 A2, auf die "Peilung" von "Blitzen", wie sie innerhalb von Wolken, zwischen diesen oder zwischen Wolken und Erde stattfinden, sondern auf die Beobachtung dieser schwachen Entladungsvorgänge, d.h. der Spherics-Burstsignale, die typischerweise innerhalb einer Wolke stattfinden. Deshalb können die Erfassungsgeräte (Sensoren) mit relativ einfachen Antennen ausgestattet sein, die aus orthogonal zueinander angeordneten Ferritstäben bestehen (alternativ könnten diese auch durch einfache Helmholtzspulen dargestellt werden). Durch die erfindungsgemäße Kombination "teure Messgeräte, geringe Zahl der Messstützpunkte" und "einfache Messgeräte/hohe Zahl der Messstützpunkte" wird auf überraschend einfache Art und Weise eine deutliche Verbesserung gegenüber bisherigen Beobachtungsmethoden erreicht. Die Aufbereitung der empfangenen Signale innerhalb der Zentrale geschieht nicht unter dem Gesichtspunkt einer physikalischen Analyse des je einzelnen Signals selbst, sondern unter dem Gesichtspunkt und der Zielsetzung, aus dem von den Sensoren "gelieferten" Ereignismustern eine "Entladungs-Wetterkarte" zusammenzustellen, die dann in Verbindung mit weiteren meteorologischen Parametern zu interpretieren ist. Somit kann das erfindungsgemäße Messnetz bzw. Verfahren in Projekten, welche einer Verbesserung der Unwetterwarnung dienen sollen, eingesetzt zu werden. Insbesondere auch in einem bundesweiten extrem engmaschigen Netz (Maschenweite <= 1km) mit deutlich vereinfachten Messgeräten für Druck, Feuchte, Temperatur, Niederschlag und Windgeschwindigkeit.

Bei einer bevorzugten Ausgestaltung der Erfindung nehmen, gemäß Patentanspruch 4, die Sensoren des Basisnetzes eine Vorverarbeitung der Spherics-Burstsignale dergestalt vor, dass das empfangene Signal nach einer A/D-Wandlung über parallel arbeitende, digitale Bandfilter geleitet wird und die in den jeweiligen Frequenzbändern sich befindenden Signale nach Impulshäufigkeit und Leistungsdichte mittels jeweils nachgeschalteten digitalen Signalprozessoren DSP analysiert werden.

Diese Ausgestaltung der Erfindung weist die Vorteile auf, dass durch die intelligenten Sensoren des Basisnetzes und durch solchermaßen gewonnene und an die Übertragungseinheit zur Weiterleitung an die Zentrale übergebene "Ereignismuster", die in der Zentrale angeordnete Auswerteeinheit kostengünstiger, einfacher und schneller ausgeführt werden kann.

Weitere Vorteile und Einzelheiten lassen sich der nachfolgenden Beschreibung einer bevorzugten Ausführungsform der Erfindung unter Bezugnahme auf die Zeichnung entnehmen. In der Zeichnung zeigt:
- FIG. 1: eine bevorzugte Ausgestaltung der Sensoren SBN des Basisnetzes BN,
- FIG. 2: eine Ausgestaltung eines Basisnetzes beim Stand der Technik,
- FIG. 3: eine bevorzugte Ausgestaltung der Sensoren SSN des Subnetzes SN,
- FIG. 4: eine schematische Darstellung des inaktiven Basisnetzes BN,
- FIG. 5: das aktive Basisnetz BN nach FIG. 4 mit vier sich selbst aktivierten Sensoren SBN,
- FIG. 6: das aktive Basisnetz BN nach FIG. 5 mit weiteren acht sich selbst aktivierten Sensoren SBN,
- FIG. 7: das aktive Basisnetz BN nach FIG. 4 mit insgesamt vierundzwanzig sich selbst aktivierten Sensoren SBN und
- FIG. 8: eine schematische Darstellung des aktiven Basisnetzes BN nach FIG. 5 und einem dem Basisnetz BN unterlagertes Subnetz SN mit Sensoren SSN gemäß der Erfindung.

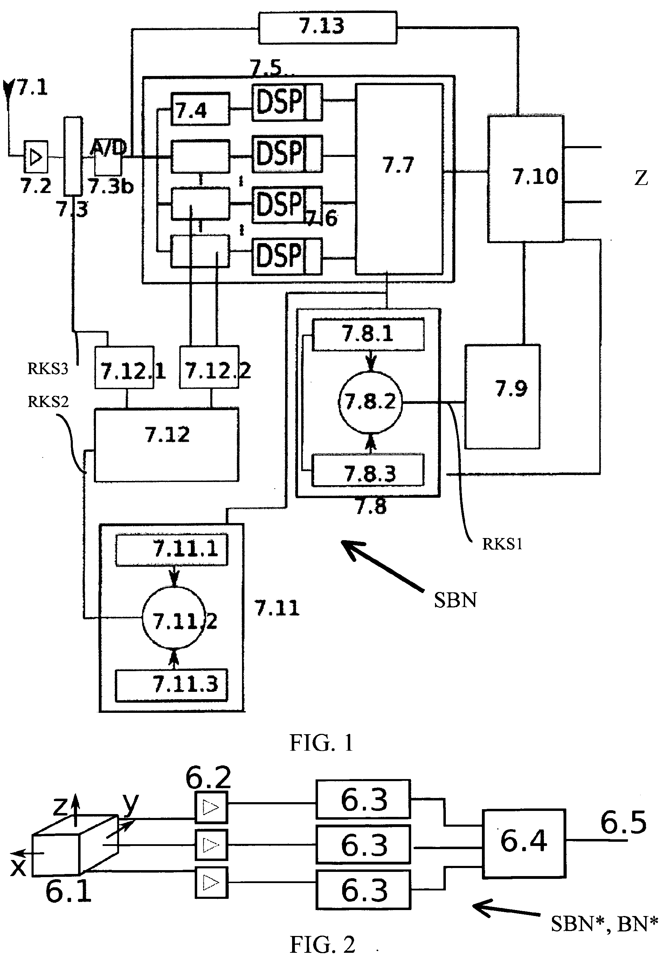

Das erfindungsgemäße Messnetz bzw. das erfindungsgemäße Verfahren zur Registrierung schwacher elektromagnetischer Signale geht von einem elektromagnetischen Messsystem für die Meteorologie gemäß der EP 0 990 177 B1 der Anmelderin mit einem Basisnetz BN* mit einer Mehrzahl von Messstationen aus, welches in FIG. 2 dargestellt ist. Die Messstationen/Sensoren SBN* zum Empfangen der elektromagnetischen Signale sind räumlich verteilt in einem vorgegebenen Raumgebiet angeordnet und weisen jeweils eine orthogonale Antenne 6.1 für x, y, z - Raumkoordinaten, in jedem Kanal (XYZ), einen Eingangsvorverstärker 6.2, eine diesem nachgeordnete Auswerteeinheit 6.3 und eine mit dieser verbundenen Übertragungseinheit 6.4 zum Übermitteln von in den Messstationen/Sensoren SBN* jeweils vorliegenden und aus den elektromagnetischen Signalen abgeleiteten Messdaten an eine den Messstationen/Sensoren SBN* zugeordnete und in einer Zentrale Z angeordneten Auswerteeinheit auf. Der Abstand jeweils benachbarter Messstationen des Basisnetzes BN* beträgt zwischen 10 km und 50 km.

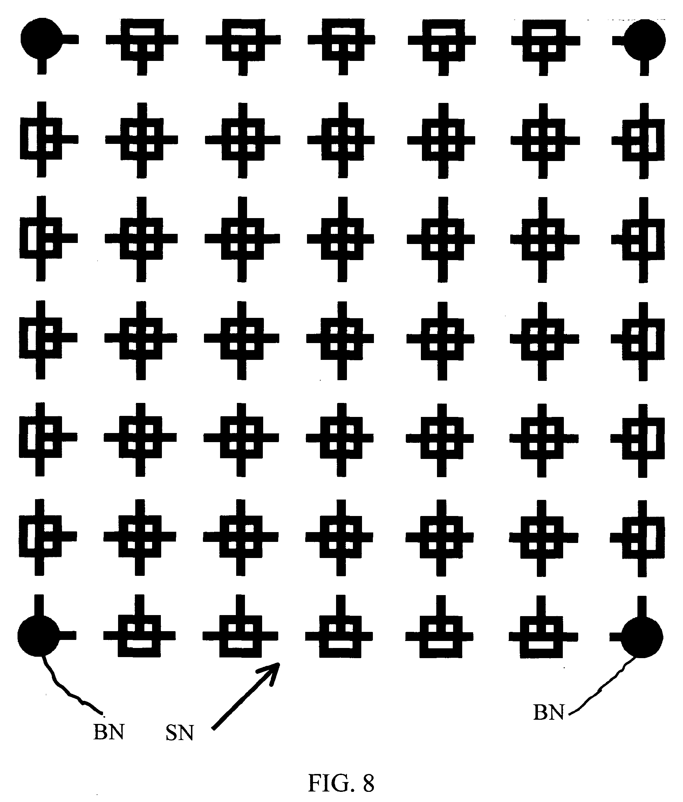

Beim erfindungsgemäßen selbstaktivierenden und adaptiven Messnetz ist dem Basisnetz BN ein Subnetz SN unterlagert (siehe FIG. 8), wobei in den Zellen des Subnetzes SN Sensoren SSN mit einem im Verhältnis zwischen 1 : 8 und 1 : 12, vorzugsweise 1 : 10, kleineren Abstand verteilt angeordnet sind. Die im Standby-Betrieb befindlichen Sensoren SBN des Basisnetzes BN, von denen eine Ausführungsform in FIG. 1 dargestellt ist, sind beim Empfang von in einem einstellbaren Zeitraum eintreffenden Spherics-Burstsignalen selbstaktivierend und nehmen eine Vorverarbeitung zur Gewinnung von Ereignismustern vor nach Maßgabe derer - gemäß der Erfindung - die Zentrale Z zur feineren Auflösung bezüglich der Beobachtung, die Sensoren SSN des Subnetzes SN einschalten kann.

Weiterhin weisen die Sensoren SBN im selbstaktivierenden und adaptiven Messnetz drei Rückkopplungsschleifen auf, nämlich eine zur Selbstaktivierung RKS1, eine zur Hinzunahme weiterer Frequenzbänder RKS2 im Sensor SBN und eine zur Absenkung des Schwellwertes RKS3 im Eingangskreis des Sensors SBN.

Im Einzelnen nehmen die Sensoren SBN des Basisnetzes BN eine Vorverarbeitung der Spherics-Burstsignale dergestalt vor, dass das empfangene Signal nach einer A/D-Wandlung 7.3.b über parallel arbeitende digitale Bandfilter 7.4 geleitet wird und die in den jeweiligen Frequenzbändern sich befindenden Signale nach Impulsrate (Häufigkeit pro Zeit) und Leistungsdichte (Amplitude) mittels jeweils nachgeschalteten digitalen Signalprozessoren DSP 7,5, 7.6 in allen drei Raumkoordinaten X, Y und Z analysiert werden. Mit den digitalen Signalprozessoren DSP 7.5, 7.6 ist eine, beispielsweise als Single-Chip- Computer mit zugehöriger Speichereinheit ausgeführte Auswerteeinheit 7.7 zur Erzeugung eines Ereignismusters verbunden. An deren Ausgang ist zur Selbstaktivierung des Sensors SBN des Basisnetzes BN ein, beispielsweise modulartig aufgebauter Vergleicher 7.8 angeordnet, wobei in dessen ersten Modul 7.8.1 das Ereignismuster zum Zeitpunkt T₁ gespeichert wird, wobei in dessen dritten Modul 7.8.3 ein sogenanntes Default-Pattern (Ereignismuster) eingeprägt ist und wobei in dessen zweites Modul 7.8.2 - bei Identität von zwei aufeinanderfolgenden Ereignismuster im ersten Modul 7.8.1 und dem im dritten Modul 7.8.3 hinterlegten Ereignismuster - der Vergleicher 7.8 ein Signal an eine Stromversorgungseinheit 7.9 für die Übertragungseinheit 7.10 liefert und diese aktiviert. Das im dritten Modul 7.8.3 hinterlegte Ereignismuster ist entweder dem Sensor SBN eingeprägt, z.B. in einem ROM, oder kann im Falle, dass die Übertragungseinheit 7.10 aktiv ist, von der Zentrale Z her geändert werden.

Der Vergleicher 7.8.2 kann entweder als "Holistischer" Vergleicher gemäß der Lehre nach DE 197 21 197 A1, d.h. eine Recheneinrichtung zum Verarbeiten einer Eingangsdatenstruktur mit einer Abbildungseinrichtung zum Abbilden der Eingangsdatenstruktur in eine mehrdimensionale analoge Bilddatenstruktur, mit einer analogen Speichereinrichtung zum Speichern der analogen Bilddatenstruktur, und mit einer Vergleichseinrichtung zum Vergleich der analogen Bilddatenstruktur mit wenigstens einer analogen Referenzdatenstruktur und zum Abbilden des Vergleichsergebnisses in eine Ausgangsdatenstruktur, in einer miniaturisierten Form ausgeführt werden, oder es erfolgt ein normaler Bitmustervergleich mithilfe eines beispielsweise Single-Chip-Computers. Die Übertragungseinheit 7.10 kann im Rahmen der Erfindung als programmierbares "Open-Platform-Handy" unter Nutzung üblicher Mobilfunknetze ausgeführt werden. Damit werden die von der Auswerteeinheit 7.7 oder einer Aufzeichnungseinheit 7.13 (im Einzelnen siehe nachfolgend) gelieferten Signale an die Zentrale Z übertragen oder von dieser ein geändertes "Default-Pattern" empfangen. Die Stromversorgung der schaltbaren Stromversorgungseinheit 7.9 selbst kann entweder über das örtliche Netz oder über Solarpanel mit Akkupufferung erfolgen.

Zur Hinzunahme weiterer Frequenzbänder und zur Variation der Eingangsempfindlichkeit ist im Sensor SBN des Basisnetzes BN ein zwei Speicher 7.11.1, 7.11.3 aufweisender Vergleicher 7.11 angeordnet, welcher mit der Auswerteeinheit 7.7 und mit einer Schalter 7.12.1; 7.12.2 aufweisenden Schalteinheit 12 verbunden ist. Falls die im ersten Speicher 7.11.1 zum Zeitpunkt Tn gespeicherten Ereignismuster zu den im zweiten Speicher 7.11.2 zum Zeitpunkt Tn+1 gespeicherten Ereignismuster unterschiedlich sind, schaltet der Vergleicher 7.11 mittels eines Schalters 7.12.2 schrittweise Frequenzbänder hinzu oder senkt mittels eines Schalters 7.12.1 den Schwellwert in einer mit der Antenne 7.1 in Verbindung stehenden Torschaltung 7.3 ab. Eine vorzugsweise zyklisch tätige Schalteinheit (Schalter) 7.12 schaltet schrittweise Frequenzbänder über die Schaltfunktionen in 7.12.2 hinzu, um insbesondere höherfrequente Frequenzanteile des empfangenen Signals auswerten zu können (bis zum VHF-Bereich) und über die Schalteinheit (Schalter) 7.12.1 wird der Schwellwert in der Torschaltung 7.3 abgesenkt. Sobald die Ereignismuster in den Speichern 7.11.1 und 7.11.3 sich nicht mehr verändern und somit kein Differenzsignal entsteht, wird dieser Vorgang beendet.

Ferner ist die Antenne 7.1 der Sensoren SBN des Basisnetzes BN mit einem Eingangsvorverstärker 7.2 und dieser mit der Torschaltung 7.3 für einen einstellbaren Schwellwert verbunden, wobei an die Torschaltung 7.3 mehrere digitale Bandfilter 7.4 (bei der in FIG. 1 dargestellten Ausführungsform vier) mit vorzugsweise 10 kHz bis 20 kHz Bandbreite angeschlossen sind und dass gemäß der Erfindung Bandfilter 7.4 (bei der in FIG. 1 dargestellten Ausführungsform sechs) als Default-Einstellung, um die Frequenzanteile des Signals, vorzugsweise zwischen Null kHz und 150 kHz (als überwiegende Frequenzanteile bei Spherics-Burstsignalen) zu verarbeiten, dienen und weitere digitale Bandfilter 7.4 schrittweise zugeschaltet werden können. Damit ist eine Erweiterung bis in den VHF-Bereich möglich.

Zur Registrierung des Echtzeitsignals weisen die Sensoren SBN des Basisnetzes BN eine Aufzeichnungseinheit 7.13 auf, welche als Single-Chip-Computer mit entsprechenden Flash-Memory ausgestaltet und so programmierbar ist, dass die aufzuzeichnende Zeit entweder fest eingestellt wird (beispielsweise zwischen 2 und 5 Minuten) oder von der Zentrale Z aus über einen Rückkanal verändert werden kann.

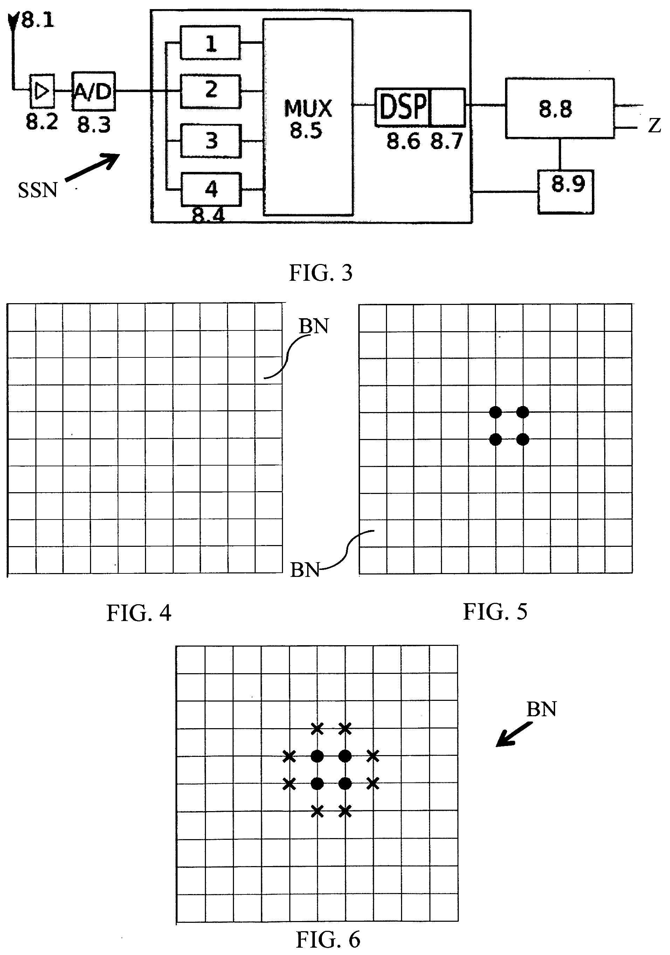

Die in FIG. 3 dargestellten Sensoren SSN des Subnetzes SN weisen keine Rückkopplungsschleifen auf. Im Einzelnen weisen die Sensoren SSN des Subnetzes SN einen mit einer Antenne 8.1 verbundenen Eingangsvorverstärker 8.2 auf, an dessen Ausgang ein A/D-Wandler 8.3 angeschlossen ist. Am Ausgang des A/D-Wandlers 8.3 sind mehrere parallel arbeitende digitale Bandfilter 8.4 (bei der in FIG. 3 dargestellten Ausführungsform vier) angeschlossen, deren Ausgänge mit einem Multiplexer 8.5 zur gemeinsamen Ansteuerung eines digitalen Signalprozessoren DSP 8.6 und einer nachgeordneten Auswerteeinheit 8.7 verbunden sind. Am Ausgang der Auswerteeinheit 8.7 ist eine Übertragungseinrichtung 8.8 angeschlossen. Im Standby-Betrieb wird nur die Übertragungseinrichtung 8.8 der Sensoren SSN des Subnetzes SN von einer Stromversorgung 8.9 mit Strom versorgt. Zur feineren Auflösung bezüglich der Beobachtung ist von der Zentrale Z aus über einen Rückkanal die Stromversorgung 8.9 auch für die vorgenannten Module Sensoren SSN einschaltbar.

Das erfindungsgemäße Verfahren zur Registrierung der Spherics-Burstsignale ist dadurch gekennzeichnet, dass die im Standby-Betrieb befindlichen Sensoren SBN des Basisnetzes BN beim Empfang von in einem einstellbaren Zeitraum eintreffenden Spherics-Burstsignalen selbstaktivierend sind, dass die Sensoren SBN des Basisnetzes BN eine Vorverarbeitung zur Gewinnung von Ereignismustern vornehmen und dass die Zentrale Z zur feineren Auflösung bezüglich der Beobachtung die Sensoren SSN des Subnetzes SN einschaltet. Die Sensoren SBN des Basisnetzes BN werden erst dann aktiviert und eine Übertragung der Information zur Zentrale Z wird erst dann ausgelöst, wenn im Zeitraum zumindest zwei Ereignismuster eintreffen, die einem vorgegebenen, durch vergangene Beobachtungen gewonnenen, entsprechen. Dabei können im Rahmen der Erfindung diese in den Sensoren SBN des Basisnetzes BN gespeicherten Ereignismuster per Fernwartung verändert werden, wenn sich im Betrieb zeigt, dass eine Veränderung des bei der Erst-Inbetriebnahme der Sensoren SBN des Basisnetzes BN nach Blitzhäufigkeit und/oder Topographie gewählten Ereignismusters sinnvoll erscheint.

Das erfindungsgemäße selbst aktivierende und adaptive Messnetz ist zusammenfassend dadurch gekennzeichnet, dass
(1) die Zahl der Messstationen SBN, SSN wesentlich erhöht wird und in die bisherige Maschenweite von ca. 30 x 30 Kilometern das Subnetz SN mit einer Maschenweite von kleiner gleich 5 Kilometer (abhängig von der Topographie) eingeschrieben wird, welches mit einfacheren Messstationen SSN bestückt wird.
(2) die Messstationen SBN sich in einem Standby-Mode befinden und erst durch die zu beobachtenden Entladungsprozesse/Spherics-Burstsignale selbst aktiviert werden. Standby-Mode bedeutet dabei, dass die Gesamtfunktionalität eines Sensors SBN erst dann aktiviert und eine Übertragung der Information zur Zentrale Z erst dann ausgelöst werden, wenn aufeinanderfolgend in einem definierten, einstellbaren Zeitraum zumindest zwei Ereignismuster eintreffen, die einem vorgegebenen, durch vergangene Beobachtungen gewonnenen, entsprechen. Diese Ereignismuster können in der Messstation SBN per Fernwartung verändert werden, wenn sich im Betrieb zeigt, dass eine Veränderung des gewählten "Default Pattern" sinnvoll erscheint. Hierzu können Funktionalitäten, wie diese in der EP 0 990 177 B1 der Anmelderin beschrieben sind, herangezogen werden.
(3) die Messstationen SBN ihrerseits sich adaptiv verhalten (oder durch Fernwartung adaptiert werden können) indem, z. B., die Station SBN automatisch
   (3.1.) den in der Grundeinstellung gewählten Frequenzbändern so lange weitere hinzugefügt werden (durch Aktivierung der zugehörigen Bandpässe im Eingang), bis die dort vorgefundenen Signale einen definierten Schwellwert nicht mehr überschreiten,
   (3.2.) bei unverändert bleibenden Frequenzbändern die Eingangsempfindlichkeit so lange erhöht wird (also der Schwellwert für die Signale in Nanotesla so lange abgesenkt wird), bis die solchermaßen beobachteten Signale nicht mehr vom "Rauschen" getrennt werden können.
(4) das Messnetz weiterhin insoweit adaptiv ist, dass die Messstationen SBN des Basisnetzes BN (siehe (1)) bei Vorliegen eines bestimmten Ereignismusters, die Messstationen SSN sowohl im Subnetz SN aktivieren, als auch benachbarte Stationen SBN des Basisnetzes BN.

Damit wird zweierlei erreicht:
- Eine wesentlich feinere Auflösung bezüglich der Beobachtung, z. B. konvektiver Zellen in der Bewölkung bezüglich ihres Entstehungsortes und ihrer Ausdehnung.
- Eine Diskriminatorfunktion, wenn z. B. zwei oder drei benachbarte Stationen SSN des Subnetzes SN (bei entsprechend gewählter hoher Eingangsempfindlichkeit) sich in ihrer Beobachtung unterscheiden. Technische Störer, z. B. Abrissfunken an elektrischen Maschinen und dergleichen, haben zwar hinsichtlich des Frequenzspektrums gewisse Ähnlichkeiten mit in der Atmosphäre durch Entladungsprozesse generierten Signalen, sind aber in ihrer Feldstärke und damit in ihrer Reichweite so gering, dass sie nicht von allen Stationen SSN des Subnetzes SN empfangen werden können. Im Umkehrschluss bedeutet dies, dass, wenn alle Stationen SSN des Subnetzes SN ein Signal registrieren, dieses mit hoher Wahrscheinlichkeit auf atmosphärische Entladungsvorgänge zurückzuführen ist.

Das erfindungsgemäße selbst aktivierende und adaptive Messnetz/Verfahren Messnetz ist also sowohl adaptiv bezüglich der Maschenweite und der Zahl der verwendeten Messstationen, als auch hinsichtlich der Eigenschaften der Messstationen selbst.

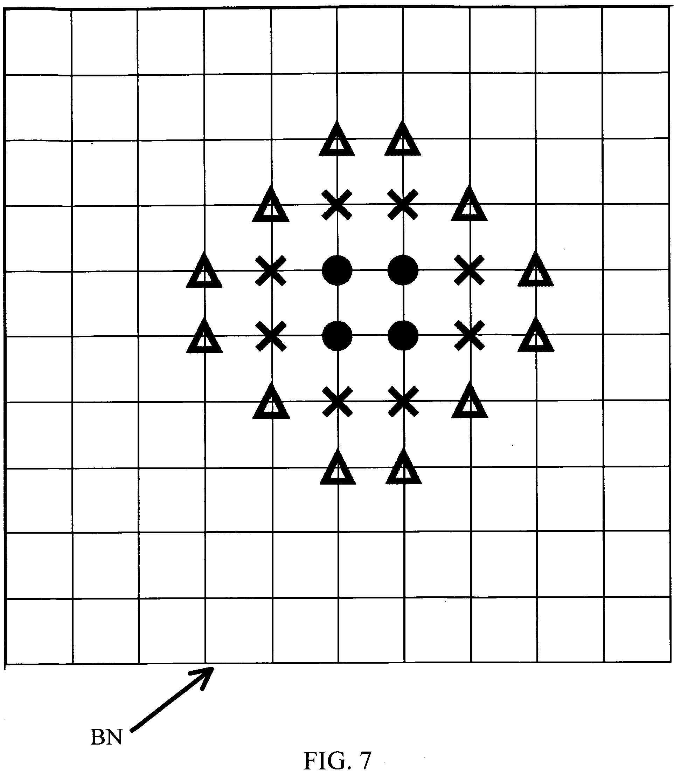

Die FIG. 4 zeigt eine schematische Darstellung des inaktiven Basisnetzes BN mit einer Maschenweite von beispielsweise 30 km. In FIG. 5 ist das aktive Basisnetz BN nach FIG. 4 mit vier sich selbst aktivierten Sensoren SBN dargestellt (siehe die vier Punkte); in FIG. 6 umfasst das aktive Basisnetz BN weitere acht sich selbst aktivierte Sensoren SBN (siehe die acht Kreuze). Weiterhin ist in FIG. 7 das aktive Basisnetz BN mit insgesamt vierundzwanzig sich selbst aktivierten Sensoren SBN dargestellt, dabei unterstellt, dass im Außenbereich keine Entladungsaktivitäten mehr stattfinden und der selbstgesteuerte Erweiterungsprozess endet. Die FIG. 8 zeigt schließlich eine schematische Darstellung des aktiven Basisnetzes BN nach FIG. 5 mit einem dem Basisnetz BN unterlagerten Subnetz SN mit Sensoren SSN, welche - gemäß der Erfindung - von der Zentrale Z aktiviert werden, sobald diese erkennt, dass eine Feinanalyse der Vorgänge notwendig ist.

Erfindungsgemäß ist das Messnetz sowohl selbstaktivierend als auch selbst-deaktivierend konzipiert. Der Deaktivierungsvorgang ist als Inversfunktion des Aktivierungsvorgangs zu verstehen. Während die Aktivierung nach dem Eintreffen von mindestens zwei als relevant erkannten Ereignismustern stattfindet, erfolgt die automatische Deaktivierung, wenn innerhalb einer einstellbaren Zeitspanne keine neuen Signale beim jeweiligen Sensor SBN des Basisnetzes BN registriert werden. Dabei entsteht folgender Ablauf: Mit Eintreten der ersten Ereignisse werden die Sensoren SBN des Basisnetzes BN aktiviert und - wie bereits beschrieben- werden die Nachbarstationen automatisch zugeschaltet. Die Gewitterzelle zieht über das gesamte Messnetz hinweg und während die zuerst aktivierten Sensoren SBN des Basisnetzes BN sich bereits wieder deaktivieren, werden gegebenenfalls weitere neue längs der Zugrichtung einer Gewitterzelle aktiviert. Nach Beendigung des Wetterereignisses geht das gesamte Netz in den Stand-By-Mode zurück. Mit Hilfe des erfindungsgemäßen Messnetzes kann räumlich und zeitlich sehr präzise die Entstehung und weitere Entwicklung eines Gewitters vorhergesagt werden. Damit eröffnen sich auch für den Blitzschutz neue Perspektiven, können doch über die Auswertung in der Zentrale Z des Messnetzes Gewitter präzise vorhergesagt werden und schon vor dem Auftreten von Blitzschlägen beim Kunden (z.B. Veranstalter von Groß- oder Sportereignissen, Flughäfen und Flugsicherung, Gartenbaubetriebe und Landwirte, Energieversorger und Windkraftbetreiber u.a.) Schutz- bzw. Vorsorgemaßnahmen eingeleitet werden. So kann beim ersten Auftreten von atmosphärischen Entladungen innerhalb eines Gebietes geprüft werden, in welche Richtung sich diese entstehende Gewitterzelle entwickeln wird. Beim Überschreiten der mit dem Kunden vereinbarten Wahrscheinlichkeit für ein Gewitter im Gebiet des Kunden wird die erste Warnung an ihn abgegeben. Die Warnmeldung selber ist immer mit dem Kunden exakt abgesprochen und erfolgt auf Basis vorhandener Nachrichtenübertragungssysteme, z. B. per E-Mail, per Fax, SMS-Nachricht auf ein Handy, Telefonanruf, Datenfernübertragung oder über das Internet. Dies ist von besonderer Wichtigkeit im Bereich der Flugmeteorologie (die Flugsicherung kann den Piloten rechtzeitig genug vor der drohenden Gefahr warnen und dieser kann das Hindernis umfliegen oder nach einer Sicherungsrunde zwar verspätet aber sicher landen) oder im Bereich der Betriebssicherheit von Rechenzentrumen (solche Rechenzentren werden in aller Regel mit einer USV-Anlage (und seltener mit einer Netzersatzanlage) betrieben) oder von Antennenmasten und Funkanlagen an exponierten Stellen (diese Anlagen können bei Gewittergefahr kurzfristig völlig außer Betrieb genommen werden, wodurch das Schadenrisiko drastisch vermindert werden kann) oder im Bereich von Sport- oder Freizeitveranstaltungen im Freien, bei denen die Veranstalter von der Tatsache in Kenntnis gesetzt werden, dass ein Gewitter im Anzug ist. Weiterhin kann aus den Wetterdaten (ähnlich der Netzlaststeuerung für die EVU's, bei der eine Steuergröße für den zu erwartenden Leistungsverbrauch innerhalb der nächsten 30 bis 90 Minuten ermittelt und an das EVU übertragen wird, so dass der Energiefluß optimiert und die Über- oder Unterversorgung mit elektrischer Energie verringert werden kann) eine zusätzliche Steuergröße für die Regelung der Heizungs- und/oder Klimaanlage abgeleitet werden, die an Tagen mit stark wechselnder Sonneneinstrahlung (Bewölkungsänderung) hilft, den Regelprozess der Heizungs- und/oder Klimaanlage zu glätten und damit Energie im Bereich zwischen 5% (Gebäude mit reiner Heiztechnik in günstiger Lage) bis über 15% (Gebäude mit Vollklimatisierung in ungünstiger Lage) einzusparen.

Die hier beschriebenen Eigenschaften des Messnetzes, nämlich ereignisgetriebene zunehmende Engmaschigkeit des Netzes bei gleichzeitig zunehmender Zahl der Beobachtungsgrößen nach Frequenzbändern und Leistungsdichteklassen, ermöglicht die Gewinnung neuer Erkenntnisse über die biotropen Wirkungen von Spherics und Spherics-ähnlichen VLF-Signalen.

In der Fachliteratur finden sich zahlreiche Veröffentlichungen, in denen signifikante bis hochsignifikante Korrelationen von Spherics- und Spherics-ähnlichen VLF-Signalen mit bestimmten Krankheitsbildern (wie beispielsweise juvenilen Epileptikern oder die Zunahme von Myokard-Infarkten) und/oder physiologischen Veränderungen beschrieben werden. Dabei zeigte sich bei der Aufzeichnung der Hirnstromkurven, dass es zu einer sogenannten Remanenz kam - die Veränderungen gingen nach der Beaufschlagung mit Spherics-Signalen nicht schlagartig, sondern langsam zurück. Die Problematik bei allen diesen Untersuchungen liegt darin, dass die jeweiligen Probandengruppen relativ klein waren, die Beobachtungszeiträume relativ kurz waren und die Untersuchungen jeweils räumlich sehr eng begrenzt waren. Bis heute konnte kein "Rezeptor" gefunden werden, über den Spherics- und Spherics-ähnliche VLF-Signale in biologische Systeme "einkoppeln". Alle veröffentlichten Untersuchungen sind demnach qualitative Berichte und keine quantifizierte Untersuchung im Sinne eines Experiments, welches sich unter gleichen Randbedingungen an allen Orten zu allen Zeiten mit identischen Resultaten wiederholen lassen muss.

Mit dem erfindungsgemäßen Messnetz ist es möglich, über ein Gebiet, z. B. der Größe der Bundesrepublik Deutschland, Aufzeichnungen über einen sehr langen Zeitraum vorzunehmen und diese Aufzeichnungen mit Krankheitsereignissen und Krankheitsverläufen einer extrem hohen Zahl von Patienten zu vergleichen. Damit entsteht zum ersten Mal eine als gesichert ansehbare statistische Basis, deren Analyse dazu beiträgt, den kausalen Ablauf der Interaktion zwischen Spherics- und Spherics-ähnlichen VLF-Signalen und biologischen Systemen zu erklären. Dies gilt auch für tektonische Ereignisse und ihr Zusammenhang mit Spherics- und Spherics-ähnlichen VLF-Signalen. In der einschlägigen Literatur finden sich zahlreiche Hinweise, dass tektonische Verschiebungen, die z. B. einem Erdbeben vorausgehen, sich ebenfalls in Spherics- und Spherics-ähnlichen VLF-Signalen abbilden. Dabei treten diese Signale unabhängig von z. B. der Entwicklung konvektiver Bewölkung, die einem Gewitter vorausgeht, aus. Als Ursache werden "Störungen" in den Wirbelströmen des Magmas vermutet, die durch relative Verschiebungen einzelner Schichten zueinander entstehen. In Konsequenz bedeutet dies, dass ein, während der Beobachtung immer engmaschiger werdendes und immer mehr Parameter erfassendes Messnetz, dessen Betrieb aufgrund der gewählten Eigenschaften kostengünstig darstellbar ist, auch eine Verbesserung im Bereich der Erdbebenprognose sowie Vulkanausbruchsvorhersage bewirkt.

Die Erfindung ist nicht auf die beschriebenen Vorgehensweisen bzw. Funktionsblöcke beschränkt, sondern umfasst auch andere Vorgehensweisen, die den gleichen erfindungswesentlichen Effekt bewirken. So kann im Rahmen der Erfindung in Abhängigkeit von der Topographie (beispielsweise Norddeutschland mit kilometerweitem flachem Gelände) anstelle einer Auswertung für die x, y, z - Raumkoordinaten (Polarisation des Quellensignals und Abschätzung der Höhenlage der Sensoren zum Quellensignal) eine Auswertung nur in XY-Richtung erfolgen oder es muss nicht in jeder Zelle des Basisnetzes BN die gleiche Anzahl von Sensoren SSN des Subnetzes SN vorgehalten werden. Ferner ist die Erfindung bislang auch noch nicht auf die in den Patentansprüchen 1 und 9 definierten Merkmalskombinationen beschränkt, sondern kann auch durch jede beliebige andere Kombination von bestimmten Merkmalen aller insgesamt offenbarten Einzelmerkmalen definiert sein. Dies bedeutet, dass jedes Einzelmerkmal des Patentanspruchs 1 oder Patentanspruchs 9 weggelassen bzw. durch mindestens ein an anderer Stelle der Anmeldung offenbartes Einzelmerkmal ersetzt werden kann.

## Patentansprüche

1. Messnetz zur Registrierung schwacher elektromagnetischer Signale mit einem Basisnetz (BN*) mit einer Mehrzahl von Messstationen, die räumlich verteilt in einem vorgegebenen Raumgebiet angeordnet sind, und die jeweils eine Antenne (6.1) und zumindest einen Sensor (SBN*) zum Empfangen der elektromagnetischen Signale und eine Übertragungseinheit (6.4) zum Übermitteln von in der Messstation jeweils vorliegenden und aus den elektromagnetischen Signalen abgeleiteten Messdaten an eine den Messstationen zugeordnete und in einer Zentrale (Z) angeordneten Auswerteeinheit umfassen wobei der Abstand jeweils benachbarter Messstationen des Basisnetzes (BN*) zwischen 10 km und 50 km beträgt, **dadurch gekennzeichnet, dass** dem Basisnetz (BN) ein Subnetz (SN) unterlagert ist, dass in Zellen des Subnetzes (SN) Sensoren (SSN) mit einem im Verhältnis zwischen 1 : 8 und 1 : 12, vorzugsweise 1 : 10, kleinerem Abstand verteilt angeordnet sind, dass im Standby-Betrieb befindliche Sensoren (SBN) des Basisnetzes (BN) beim Empfang von in einem einstellbaren Zeitraum eintreffenden Spherics-Burstsignalen selbstaktivierend sind und eine Vorverarbeitung zur Gewinnung von Ereignismustern vornehmen und dass die Zentrale (Z) zur feineren Auflösung bezüglich der Beobachtung, die Sensoren (SSN) des Subnetzes (SN) einschaltet.

2. Messnetz nach Anspruch 1, **dadurch gekennzeichnet, dass** die Sensoren (SBN) des Basisnetzes (BN) drei Rückkopplungsschleifen aufweisen, nämlich eine zur Selbstaktivierung (RKS1), eine zur Hinzunahme weiterer Frequenzbänder (RKS2) im Sensor (SBN) und eine zur Absenkung des Schwellwertes (RKS3) im Eingangskreis des Sensors (SBN) und dass die Sensoren (SSN) des Subnetzes (SN) keine Rückkopplungsschleifen aufweisen.

3. Messnetz nach Anspruch 1, **dadurch gekennzeichnet, dass** die Antenne (7.1) der Sensoren (SBN) des Basisnetzes (BN) mit einem Eingangsvorverstärker (7.2) und dieser mit einer Torschaltung (7.3) für einen einstellbaren Schwellwert verbunden ist, dass mit der Torschaltung (7.3) mehrere digitale Bandfilter (7.4) mit 10 kHz bis 20 kHz Bandbreite angeschlossen sind und dass ein Teil der Bandfilter (7.4) zur Default-Einstellung, um die Frequenzanteile des Signals zwischen Null kHz und 150 kHz zu verarbeiten, dienen und weitere digitale Bandfilter (7.4) schrittweise zugeschaltet werden können.

4. Messnetz nach Anspruch 1, **dadurch gekennzeichnet, dass** die Sensoren (SBN) des Basisnetzes (BN) eine Vorverarbeitung der Spherics-Burstsignale dergestalt vornehmen, dass das empfangene Signal nach einer A/D-Wandlung (7.3.b) über parallel arbeitende, digitale Bandfilter (7.4) geleitet wird und die in den jeweiligen Frequenzbändern sich befindenden Signale nach Impulshäufigkeit und Leistungsdichte mittels jeweils nachgeschalteten digitalen Signalprozessoren (DSP; 7,5, 7.6) analysiert werden.

5. Messnetz nach Anspruch 4, **dadurch gekennzeichnet, dass** mit den digitalen Signalprozessoren (DSP; 7.5, 7.6) eine als Single-Chip- Computer mit zugehöriger Speichereinheit ausgeführte Auswerteeinheit (7.7) zur Erzeugung eines Ereignismusters verbunden ist und dass an deren Ausgang zur Selbstaktivierung des Sensors (SBN) des Basisnetzes (BN) ein modulartig aufgebauter Vergleicher (7.8) angeordnet ist, wobei in dessen erstem Modul (7.8.1) das Ereignismuster zum Zeitpunkt T₁ gespeichert wird, wobei in dessen drittem Modul (7.8.3) ein sogenanntes Default-Pattern eingeprägt ist und wobei dessen zweites Modul (7.8.2) - bei Identität von zwei aufeinanderfolgenden Ereignismuster der von im ersten Modul (7.8.1) und dem im dritten Modul (7.8.3) hinterlegten Ereignismuster - ein Signal an eine Stromversorgungseinheit (7.9) für die Übertragungseinheit (7.10) liefert und diese aktiviert.

6. Messnetz nach Anspruch 5, **dadurch gekennzeichnet, dass** zur Hinzunahme weiterer Frequenzbänder und zur Variation der Eingangsempfindlichkeit ein zwei Speicher (7.11.1, 7.11.3) aufweisender Vergleicher (7.11) mit der Auswerteeinheit (7.7) und mit einer mehrere Schalter (7.12.1; 7.12.2) aufweisenden Schalteinheit (12) verbunden ist und dass der Vergleicher (7.11), falls die im ersten Speicher (7.11.1) zum Zeitpunkt Tn gespeicherten Ereignismuster zu den im zweiten Speicher (7.11.2) zum Zeitpunkt Tn+1 gespeicherten Ereignismustern unterschiedlich sind, mittels einem Schalter (7.12.2) schrittweise Frequenzbänder hinzuschaltet oder mittels einem Schalter (7.12.1) den Schwellwert in einer mit der Antenne (7.1) in Verbindung stehenden Torschaltung (7.3) absenkt.

7. Messnetz nach Anspruch 1, **dadurch gekennzeichnet, dass** zur Registrierung des Echtzeitsignales die Sensoren (SBN) des Basisnetzes (BN) eine Aufzeichnungseinheit (7.13) aufweisen, welche als Single-Chip-Computer mit entsprechenden Flash-Memory ausgestaltet und so programmierbar ist, dass die aufzuzeichnende Zeit entweder fest eingestellt wird oder von der Zentrale (Z) aus über den Rückkanal verändert werden kann.

8. Messnetz nach Anspruch 1, **dadurch gekennzeichnet, dass** die Sensoren (SSN) des Subnetzes (SN) einen mit einer Antenne (8.1) verbundenen Eingangsvorverstärker (8.2) aufweisen, an dessen Ausgang ein A/D-Wandler (8.3) angeschlossen ist, dass am Ausgang des A/D-Wandlers (8.3) mehrere parallel arbeitende digitale Bandfilter (8.4) angeschlossen sind, deren Ausgänge mit einem Multiplexer (8.5) zur gemeinsamen Ansteuerung eines digitalen Signalprozessors DSP (8.6) und einer nachgeordneten Auswerteeinheit (8.7) verbunden sind, dass am Ausgang der Auswerteeinheit (8.7) eine Übertragungseinrichtung (8.8) angeschlossen ist, welche im Standby-Betrieb der Sensoren (SSN) des Subnetzes (SN) von einer Stromversorgung (8.9) mit Strom versorgt wird und dass von der Zentrale (Z) aus über den Rückkanal die Stromversorgung (8.9) auch für die vorgenannten Module einschaltbar ist.

9. Verfahren zur Registrierung schwacher elektromagnetischer Signale in einem Messnetz mit einem Basisnetz (BN) mit Sensoren (SBN) und einem dem Basisnetz (BN) unterlagerten Subnetz (SN) mit Sensoren (SSN), gemäß einem der Ansprüche 1 bis 8, bei dem
• die im Standby-Betrieb befindlichen Sensoren (SBN) des Basisnetzes (BN) beim Empfang von in einem einstellbaren Zeitraum eintreffenden Spherics-Burstsignalen selbstaktivierend sind,
• die Sensoren (SBN) des Basisnetzes (BN) eine Vorverarbeitung zur Gewinnung von Ereignismustern vornehmen und
• die Zentrale (Z) zur feineren Auflösung bezüglich der Beobachtung die Sensoren (SSN) des Subnetzes (SN) einschaltet.

10. Verfahren nach Anspruch 9, **dadurch gekennzeichnet, dass** die Sensoren (SBN) des Basisnetzes (BN) erst dann aktiviert und eine Übertragung der Information zur Zentrale (Z) erst dann ausgelöst wird, wenn im Zeitraum zumindest zwei Ereignismuster eintreffen, die einem vorgegebenen, durch vergangene Beobachtungen gewonnenen Ereignismuster entsprechen und dass diese in den Sensoren (SBN) des Basisnetzes (BN) gespeicherten Ereignismuster per Fernwartung verändert werden können, wenn sich im Betrieb zeigt, dass eine Veränderung eines bei der Erst-Inbetriebnahme der Sensoren (SBN) des Basisnetzes (BN) nach Blitzhäufigkeit und/oder Topographie gewählten Ereignismusters sinnvoll erscheint.

11. Verfahren nach Anspruch 9, **dadurch gekennzeichnet, dass** eine automatische Deaktivierung von Sensoren (SBN) des Basisnetzes (BN) erfolgt, wenn innerhalb einer vorgegebenen oder einstellbaren Zeitspanne keine neuen Signale beim jeweiligen Sensor (SBN) des Basisnetzes (BN) registriert werden.

12. Verfahren nach Anspruch 9, **dadurch gekennzeichnet, dass** in Abhängigkeit von der Topographie anstelle einer Auswertung für die x, y, z -Raumkoordinaten zur Bestimmung von Polarisation des Quellensignals und Abschätzung der Höhenlage der Sensoren (SBN, SSN) zum Quellensignal eine Auswertung nur in XY-Richtung erfolgt.

13. Verfahren nach Anspruch 9, **dadurch gekennzeichnet, dass** in Abhängigkeit von der Topographie nicht in jeder Zelle des Basisnetzes (BN) die gleiche Anzahl von Sensoren (SSN) des Subnetzes (SN) vorgehalten wird.

14. Verfahren nach Anspruch 9, **gekennzeichnet durch** die Anwendung im Bereich der Landwirtschaft, Verkehrstelematik, Blitzschutztechnik, Flugmeteorologie, Erdbebenprognose sowie Vulkanausbruchsvorhersage und Medizintechnik.

## Claims

1. A measuring network for registering weak electromagnetic signals comprising a base network (BN *) with a plurality of measuring stations, which are distributed spatially in a predetermined spatial region, and each having an antenna (6.1) and at least one sensor (SBN*) for receiving the electromagnetic signals, and a transmission unit (6.4) for transmitting present measurement data from each measuring station to an evaluation unit arranged in a control center (Z), said present measurement data being derived from the electromagnetic signals, and, wherein the distance between each adjacent measuring station of the basic network (BN *) is between 10 km and 50 km, **characterized in that** a subnetwork (SN) is subordinated to the base network (BN), wherein subnetwork sensors (SSN) are distributed in cells of the subnetwork (SN) in a smaller distance with a ratio of between 1: 8 and 1: 12, preferably 1: 10, wherein the sensors (SBN) located in the basic network (BN), which are in standby-mode and which if receiving Spherics-burst signals in an adjustable time period are self-activating and make a preprocessing for the extraction of event patterns, and wherein the control center (Z) turns on said subnetwork sensors (SSN) a finer resolution with respect to an observation.

2. The measuring network according to claim 1, **characterized in that** the sensors (SBN) of the base network (BN) have three feedback loops, namely, one for self-activating (RKS 1), one for the addition of further bands (RKS2) in the sensor (SBN) and one for lowering the threshold value (RKS3) in the input circuit of the sensor (SBN) and wherein the subnetwork sensors (SSN) do not have feedback loops.

3. The measuring network according to claim 1, **characterized in that** the antenna (7.1) of the sensors (SBN) of the base network (BN) is connected to an input preamplifier (7.2), which is connected to a gate circuit (7.3) for an adjustable threshold value, wherein several digital band filters (7.4) filters with 10 kHz to 20 kHz bandwidth are connected to the gate (7.3) and wherein a part of the band-pass filters (7.4) serve to the default setting, for processing the frequency components of the signal between zero kHz and 150 kHz, and other digital band filters (7.4) can be switched on gradually.

4. The measuring network according to claim 1, **characterized in that** the sensors (SBN) of the base network (BN) makes a preprocessing of the Spherics-burst signals in such a way, that the received signal after an A/D-conversion (7.3.b) are lead over digital band-pass filter (7.4) working in parallel and the pulse signals located at the ends of the respective frequency bands are in each case analyzed by downstream connected digital signal processors (DSP, 7.5, 7.6) in accordance with frequency of the pulses and power density.

5. The measuring network according to claim 4, **characterized in that** a single-chip computer with associated memory unit working as evaluating unit (7.7) is connected to the digital signal processors (DSP, 7.5, 7.6) for generating an event pattern, and wherein at the output of said evaluating unit (7.7) for self-activating of the sensor (SBN) of the base network (BN) a modular comparator (7.8) is arranged, wherein in a first module (7.8.1) of said modular comparator (7.8) the event sample at time T1 is stored, wherein in a third module (7.8.3) a so-called default pattern is impressed and wherein a second module (7.8.2) - in the case of identity of two successive event patterns stored in the first module (7.8.1) and the third module (7.8.3) - delivers a signal to a power supply unit (7.9) determined for a transfer unit (10.7) and activates them.

6. The measuring network according to claim 5, **characterized in that** for the addition of further frequency bands and for the variation of the input sensitivity a two-memory (7.1 1.1, 7.1 1.3) comprising a comparator (7.1 1) that is connected to the evaluation unit (7.7) and to a switching unit (12) having a plurality of switches (7.12.1, 7.12.2) and wherein, if the event patterns stored in the first memory (7.1 1.1) at a time Tn +1 are different from the event patterns stored in the second memory (7.1 1.2) at time Tn, the comparator (7.1 1) by means of a switch (7.12.2) gradually added on frequency bands by means of a switch (7.12.1) or lowers the threshold in a gate circuit (7.3) to be connected with the antenna (7.1).

7. The measuring network according to claim 1, **characterized in that** for registration of the real-time signal, the sensors (SBN) of the basic network (BN) have a recording unit (7.13), which is designed as a single-chip computer with a corresponding flash memory and is programmable so that the recorded time is to be set manually or can be changed by the control center (Z) via the return channel.

8. The measuring network according to claim 1, **characterized in that** the subnetwork sensors (SSN) comprise an input preamplifier (8.2) connected to an antenna (8.1), wherein at output of the preamplifier (8.2) an A/D converter (8.3) is connected, wherein at the output of the A/D converter (8.3) a plurality of parallel-operating digital band-pass filter (8.4) are connected, the outputs of which for the joint control of a digital signal processor DSP (8.6) are connected to a multiplexer (8.5) and a serial connected evaluation unit (8.7) wherein at the output of the evaluation (8.7) a transmission device (8.8) is connected, which in standby operation of the subnetwork sensors (SSN) is supplied with power by a power supply (8.9) and wherein by the return channel the power supply (8.9) for the aforementioned modules can be switched on by the control center (Z).

9. A method for registering weak electromagnetic signals in a measurement system comprising a base network (BN) having sensors (SBN) and a subnet (SN) with subnetwork sensors (SSN) subordinated to the base network (BN) according to any one of claims 1 to 8, wherein
• if receiving Spherics-burst signals incoming in an adjustable time period, the mode sensors (SBN) of the basic network (BN) that are in standby-mode are self-activating,
• a pre-processing for creating event patterns is performed by the sensors (SBN) of the basic network (BN) and
• for finer resolution with respect to the observation the control center (Z) turns on the subnetwork sensors (SSN).

10. The method according to claim 9, **characterized in that** the sensors (SBN) of based network (BN) only be activated and transferring the information to the control center (Z) if in said period at least two event patterns are arriving and matching to a given pattern, which is gained by observations through past, and wherein these in the sensors (SBN) of the based network (BN) stored event pattern can be changed remotely if during operation a change of an event pattern selected to flash frequency and/or topography during the initial start-up of the sensors (SBN) of the basic network (BN) sufficiently correlates.

11. The method according to claim 9, **characterized in that** the automatic deactivation of sensors (SBN) of the base network (BN) is carried out, if in a predetermined or adjustable period of time any new signals in the respective sensor (SBN) of the base network (BN) is registered.

12. The method according to claim 9, **characterized in that** according to the topography for determining the polarization of the source signal and estimating the height position of the sensors (SBN, SSN) of the base network and subnetwork sensors in relation to a source signal instead of an evaluation of the x, y, z-space coordinates only an evaluation in the XY-direction is used.

13. The method according to claim 9, **characterized in that** according to the topography not in each cell of the base network (BN) the same number of subnetwork sensors (SSN) is maintained.

14. The method according to claim 9, **wherein** the method is utilized to make observations in a field of agriculture, transport telematics, lightning protection systems, aviation meteorology, earthquake prediction as well as prediction of volcanic eruption and medical technology.

## Revendications

1. Un réseau de mesure pour l'enregistrement des signaux électromagnétiques faibles avec un réseau de base (BN*) ayant une pluralité de stations, qui sont réparties spatialement disposés dans une région prédéterminée de l'espace, et ayant chacune une antenne (6.1) et au moins un capteur (SBN*) pour la réception de signaux électromagnétique et une unité de transfert (6.4) pour associé à la transmission des données de mesure provenant d'une station de mesure et dérivée des signaux électromagnétiques disposé à la station de mesure à une unité d'évaluation dans un centre de contrôle (Z), dans lequel la distance entre des stations de mesure respectivement avoisinantes du réseau de base (BN*) est entre 10 kilomètres et 50 kilomètres, **caractérisé en ce qu'** un sous-réseau (SN) est intégré dans le réseau de base (BN), lequel possède dans les cellules du sous-réseau (SN) des capteurs (SSN) installés à une distance plus petite d'un rapport entre 1 : 8 et 1 : 12, de préférence de 1 : 10, que les capteurs (SBN) du réseau de base (BN), étant en fonctionnement Standby, sont auto-activant, en captant des signaux Sferics-Burst arrivants dans un délai réglable et de faire un prétraitement pour avoir des modèle d'événements est effectué et que la station centrale (Z) met en marche les capteurs (SSN) du sous-réseau (SN) pour plus fine résolution par rapport à l'observation.

2. Le réseau de mesure, selon la revendication 1, **caractérisé en ce que** les capteurs (SBN) du réseau de base (BN) possèdent trois boucles de rétroaction, c'est-à-dire, une pour l'auto-activation (RKS1), une pour l'ajout d'autres bandes de fréquences (RKS2) dans le capteur (SBN) et une pour l'abaissement de la valeur de seuil (RKS3) dans le circuit d'entrée du capteur (SBN) et que les capteurs (SSN) du sous-réseau (SN) ne pas avoir des boucles de rétroaction.

3. Le réseau de mesure, selon la revendication 1, **caractérisé en ce que** l'antenne (7.1) des capteurs (SBN) du réseau de base (BN) est reliée avec un préamplificateur (7.2) et ceci avec un circuit de porte (7.3) pour un seuil réglable, qu'avec le circuit de porte (7.3) plusieurs filtres passe-bandes numériques (7.4) avec une bande passante de 10 kHz à 20 kHz sont connectée et que de la partie du filtres passe-bandes (7.4) ayant un réglage par défaut, servent pour traiter les composantes de fréquences des signaux entre zéro et 150 kHz et que d'autres filtres passe-bandes (7.4) numériques peut être commutée de façon graduelle.

4. Le réseau de mesure, selon la revendication 1, **caractérisé en ce que** les capteurs (SBN) du réseau de base (BN) effectuent un prétraitement des signaux Sferics-Burst de telle manière que le signal reçu après une conversion A/D (7.3.b) passe par un filtre passe-bande numérique (7.4) fonctionnant en parallèle et on analyse les signaux, étant dans la bande de fréquences respective pour avoir la fréquence d'impulsions et la densité de puissance à l'aide des processeurs de signaux numériques DSP (7.5, 7.6) étant respectivement connectés en aval.

5. Le réseau de mesure, selon le revendication 4, **caractérisé en ce que** avec les processeurs de signaux numériques (DSP ; 7.5, 7.6) une unité d'évaluation (7.7) est connectée, constitué comme Single-Chip-Computer, avec une unité de mémoire appartenant pour la génération d'un modèle d'événements et que à la sortie de celui un comparateur (7.8) d'une structure modulaire est installé pour l'auto-activation des capteurs (SBN) du réseau de base (BN) où on mémorise dans le premier module (7.8.1) le modèle d'événements pour le temps T₁, où dans lequel le troisième module (7.8.3) est mémorisé un soit disant Default-Pattern, où dans le deuxième module (7.8.2) s'il y a une identité de deux modèles d'événements suivants dans le premier module (7.8.1) et dans le troisième module (7.8.3) ayant mémorisé le modèle d'événement, dans ce cas le comparateur (7.8) fourni un signal à l'alimentation électrique (7.9) pour l'unité de transfert (7.10) et les active.

6. Le réseau de mesure, selon le revendication 5, **caractérisé en ce que** pour ajouter d'autres bandes de fréquences et pour faire varier la sensibilité d'entrée un comparateur (7.11) possédant deux mémoires (7.11.1, 7.11.3) et une unité de commutation (12) possédant une unité d'évaluation (7.7) et deux commutateurs (7.12.1, 7.12.2) sont connectés et que le comparateur (7.11), si le modèle d'événements mémorisé dans la première mémoire (7.11.1) enregistrés de temps Tn sont différents du modèle d'événement mémorisé dans la deuxième mémoire (7.11.2) au temps Tn+1, connecte progressivement à l'aide d'un commutateur (7.12.2) des bandes de fréquences ou à l'aide d'un commutateur (7.12.1) abaisse le seuil à l'antenne (7.1) reliée avec un circuit de porte (7.3).

7. Le réseau de mesure, selon le revendication 1, **caractérisé en ce que** pour l'enregistrement des signaux en temps réel, les capteurs (SBN) du réseau de base (BN) ayant une unité d'enregistrement (7.13), qui est réalisé comme un ordinateur mono-puce à mémoire flash correspondant et peut être programmé de telle sorte que le temps enregistré doit être réglé à une valeur fixe ou il peut être modifiée via d'un canal de retour par la station centrale (Z).

8. Le réseau de mesure, selon le revendication 1, **caractérisé en ce que** les capteurs (SSN) du sous-réseau (SN) ayant un amplificateur d'entrée, étant connecté à une antenne (8.1) et à son sortie un convertisseur A/D (8.3) est connecté, que à la sortie du convertisseur (8.3) plusieurs filtres passe-bandes numériques sont connectés fonctionnant en parallèle, leur sorties sont connectées avec un multiplexeur (8.5) pour une commande commune d'un processeur de signaux numérique DSP (8.6) et une unité d'évaluation (8.7) installé en aval, que à la sortie de l'unité d'évaluation (8.7) une unité de transmission (8.8) est connecté, laquelle est alimentée, si le capteurs (SSN) du sous-réseau (SN) étant en fonctionnement Standby, par une alimentation électrique (8.9), et qu'on peut mettre en marche l'alimentation électrique aussi pour les module mentionnés ci-dessus de la station centrale (Z) via le canal de retour.

9. Procédé pour l'enregistrement des signaux électromagnétiques faibles dans un système de mesure avec un réseau de base (BN) avec des capteurs (SBN) et le réseau de base (BN) de sous-réseau secondaire (SN) avec des capteurs (NSS) selon l'une des revendications 1 à 8, dans lequel
• l'situé dans les capteurs de mode veille (SBN) du réseau de base (BN) lors de la réception d'entrée sur un intervalle Spherics burst signaux sont auto-activation,
• les capteurs (SBN) du réseau de base (BN) fait un prétraitement pour l'obtention de modèles d'événements et
• pour une résolution plus fine par rapport la station centrale (Z) mis en marche les capteurs d'observation (SSN) du sous-réseau (SN).

10. Procédé selon la revendication 9, **caractérisé en ce que** les capteurs (SBN) du réseau de base (BN) n'est activées et une transmission de l'information à la centrale de commande (Z) est uniquement déclenchée que, dans la période de temps, au moins arriver deux motifs d'événements, l'une prédéterminée modèles d'événements correspondants obtenus par des observations passées et que celles-ci modèles d'événements stockés dans les capteurs (SBN) du réseau de base (BN) peuvent être modifiés à distance lors de l'affichage de l'opération qu'un changement d'au démarrage initial des capteurs (SBN) de réseau basé (BN) élu par la fréquence du flash et / ou motif d'événement de la topographie est logique.

11. Procédé selon la revendication 9, **caractérisé en ce qu**'une désactivation automatique des capteurs (SBN) du réseau de base (BN) est effectuée si, dans une période prédéterminée ou ajustable de temps, aucun nouveau signal au capteur respectif (SBN) du réseau de base (BN) sont enregistrés.

12. Procédé selon la revendication 9, **caractérisé en ce que** en fonction de la topographie au lieu d'une évaluation pour les coordonnées x, y, z - pour la détermination de la polarisation du signal de source et d'estimer la position en hauteur des capteurs (SBN, NSS) de la source du signal, une l'évaluation dans la direction XY seulement est réalisée.

13. Procédé selon la revendication 9, **caractérisé en ce que** en fonction de la topographie non dans chaque cellule du réseau de base (BN), le même nombre de capteurs (SSN) du sous-réseau (SN) est maintenu.

14. Procédé selon la revendication 9, **caractérisé par** l'application dans le domaine de la agriculture, la télématique des transports, de l'équipement de protection contre la foudre, la météorologie aéronautique, la prévision des tremblements de terre et de prévision de l'éruption volcanique et médicales.
